# EUROPÄISCHE PATENTSCHRIFT

(11) **EP 2 417 093 B1**
(45) Veröffentlichungstag und Bekanntmachung des Hinweises auf die Patenterteilung: **22.01.2014**
(21) Anmeldenummer: 10709807.1
(22) Anmeldetag: 18.03.2010
(51) Int. Cl.: C07C 45/50, C07C 47/02

(54) **VERFARHEN ZUR HERSTELLUNG VON ALDEHYDEN**
METHOD FOR PRODUCING ALDEHYDES
PROCÉDÉ DE PRÉPARATION D'ALDÉHYDES

(30) Priorität: 07.04.2009 DE 102009016651
(43) Veröffentlichungstag der Anmeldung: 15.02.2012
(73) Patentinhaber: OXEA GmbH, 46147 Oberhausen (DE)
(72) Erfinder: GREB, Wolfgang, 46535 Dinslaken (DE); LUKAS, Rainer, 45133 Essen (DE); SCHMID, Klaus, 46539 Dinslaken (DE); ZGORZELSKI, Wolfgang, 46149 Oberhausen (DE)
(86) Internationale Anmeldenummer: PCT/EP2010/001712
(87) Internationale Veröffentlichungsnummer: WO 2010/115509

(56) Entgegenhaltungen:
- EP-A1- 0 805 138
- EP-A1- 1 193 239

## Beschreibung

Die Erfindung betrifft ein verbessertes Verfahren zur Hydroformylierung olefinisch ungesättigter Verbindungen in Gegenwart einer wässrigen, wasserlösliche Rhodium-Komplexverbindungen enthaltenden Katalysatorlösung und insbesondere die Nutzung der mit dem Abgas aus der Hydroformylierungszone entweichenden, nicht umgesetzten olefinisch ungesättigten Verbindungen.

Es ist bekannt, dass Verbindungen, die olefinische Doppelbindungen enthalten, mit Kohlenmonoxid und Wasserstoff zu Aldehyden umsetzbar sind (Oxosynthese). Der Prozess ist nicht auf den Einsatz olefinischer Kohlenwasserstoffe begrenzt, sondern erstreckt sich auch auf Ausgangsstoffe, die außer der Doppelbindung noch funktionelle Gruppen aufweisen, vorwiegend solche, die unter den Reaktionsbedingungen unverändert bleiben.

Die klassische Oxosynthese arbeitet mit Kobalt als Katalysator. Seine Wirksamkeit beruht auf der Bildung von Kobaltcarbonylverbindungen unter der Einwirkung von Wasserstoff und Kohlenmonoxid bei Drücken oberhalb 20 MPa und Temperaturen von etwa 120°C und mehr auf metallisches Kobalt oder Kobaltverbindungen.

Im Laufe der Weiterentwicklung der Oxosynthese wurde Kobalt zunehmend durch Rhodium als Katalysatormetall ersetzt. Rhodium wird als Komplexverbindung eingesetzt, das neben Kohlenmonoxid vorzugsweise Phosphine als Liganden enthält. Rhodium als Metall erlaubt es, bei niedrigen Drücken zu arbeiten, überdies erzielt man höhere Ausbeuten und bevorzugt werden die für die Weiterverarbeitung wertvolleren unverzweigten Produkte gebildet, wenn man von geradkettigen endständigen Olefinen ausgeht.

Eine weitere technische Entwicklung der Oxosynthese bedeutete der Übergang vom homogenen zum zweiphasigen Reaktionsmedium, d.h. im Einsatzmaterial und im Rohprodukt gelösten Katalysatoren zu wässrigen Katalysatorlösungen, die als eigene Phase getrennt von Einsatzstoffen und Reaktionsprodukten vorliegen. Diese Variante der Oxosynthese ist z.B. aus DE-B-26 27 354 bekannt. Ihr besonderer Vorteil ist die leichte Trennung von Reaktionsprodukt und Katalysator, die schonend ohne Anwendung thermischer Verfahrensschritte erfolgt und daher Verluste vermeidet, die durch Folgereaktionen der entstandenen Aldehyde eintreten. Weiterhin erzielt man sehr hohe Ausbeuten und bei der Verwendung unverzweigter endständstänzeiger Olefine erhält man ganz überwiegend n-Aldehyde. Diese Verfahrensvariante wird auch als heterogenes oder zweiphasiges Verfahren bezeichnet.

Aus Gründen der Prozessökonomie, insbesondere um große Reaktoren oder lange Reaktionszeiten zu vermeiden, führt man die Umsetzung nicht bis zum vollständigen Verbrauch der olefinisch ungesättigten Verbindungen, sondern begnügt sich häufig mit der Umwandlung von lediglich 60 bis 95 % des Ausgangsmaterials zur gewünschten Endverbindung. Im Abgas, das die Hydroformylierungszone verlässt, befinden sich daher neben überschüssigem Kohlenmonoxid und Wasserstoff nicht umgesetztes olefinisches Einsatzmaterial, das nach unterschiedlich ausgestalteten Verfahren in Wertstoffe umgewandelt werden kann.

Gemäß EP-B1-0 111 257 setzt man Abgas aus einer ersten Hydroformylierungsstufe, in der Olefin mit Kohlenmonoxid und Wasserstoff nach dem zweiphasigen Hydroformylierungsverfahren umgesetzt werden, in einer zweiten Stufe nach dem klassischen Oxoverfahren bei hohem Druck in Gegenwart von Kobaltkatalysatoren um.

Eine Weiterentwicklung dieses Prozesses ist aus EP 0 805 138 A1 bekannt, in dem das Abgas der ersten, nach heterogener Fahrweise durchgeführten Hydroformylierungsreaktion in einer zweiten Stufe in einem homogenen Reaktionssystem in Gegenwart von Komplexverbindungen des Rhodiums mit organischen Phosphor(III)-Verbindungen als Katalysatoren umgesetzt wird.

Dieses Verfahren ermöglicht, dass der größte Teil der im Abgas enthaltenen, in der ersten Stufe nicht umgesetzten olefinisch ungesättigten Verbindung hydroformyliert wird. Die Vorteile dieses Prozesses waren nicht vorherzusehen. Zum einen liegen die olefinisch ungesättigten Verbindungen im Abgas in beträchtlicher Verdünnung vor, etwa mit einem Gehalt von 20 bis 70 Gew.-%. Derartige Konzentrationen stehen bei dieser Reaktionsart einer weitgehenden Umsetzung der ungesättigten Ausgangsverbindung entgegen. Ebenfalls werden mit dem Abgas aus der ersten Reaktionsstufe Verunreinigungen ausgetragen, beispielsweise schwefelhaltige Abbauprodukte des Katalysatorsystems wie Mercaptane sowie Wasser, dem Lösungsmittel für den Katalysator in der ersten Stufe. Sowohl die in dem organischen Medium der zweiten, homogen durchgeführten Reaktionsstufe gut löslichen Mercaptane und weitere Verunreinigungen können den homogen gelösten Rhodiumkomplexkatalysator schädigen und zur Bildung von katalytisch inaktiven Verbindungen führen. Trotz dieser zunächst erwarteten Nachteile kann der aus EP 0 805 138 A1 bekannte Prozess mit hoher Effizienz durchgeführt werden.

Ein charakteristisches Merkmal der aus EP 0 805 138 A1 bekannten zweistufigen Verfahrensführung ist die Wahl des Druckbereichs in der zweiten, homogen durchgeführten Hydroformylierungsstufe. Die einzuhaltenden Drücke liegen im Bereich von 15 bis 35 MPa und sind ungewöhnlich für ein homogenes Hydroformylierungsverfahren in Gegenwart von Komplexverbindungen des Rhodiums mit organischen Phosphorverbindungen. Das Umsetzungsprodukt aus dieser, bei vergleichsweise hohen Drücken durchgeführten zweiten Reaktionsstufe wird vom Katalysator abdestilliert. Der verbleibende, Katalysator enthaltende Destillationsrückstand wird gegebenenfalls nach Zusatz von Frischkatalysator und Entnahme eines Teils der im Verlauf der Reaktion gebildeten hochsiedenden Aldehydkondensationsprodukte in die Reaktionszone rezirkuliert.

Bei diesen hochsiedenden Aldehydkondensationsprodukten handelt es sich um ein komplexes Gemisch aus sauerstoffhaltigen Verbindungen, die aus den zunächst gebildeten Aldehyden durch Kondensationsreaktionen, wie der Aldolreaktion, oder durch die Tischtschenko-Reaktion, entstehen und einen unterschiedlichen Kondensationsgrad aufweisen.
Die Bildung von beispielsweise Dimeren, Trimeren oder Tetrameren als hochsiedende Aldehydkondensationsprodukte bei der homogen durchgeführten Hydroformylierungsreaktion wird beispielsweise in US 4,247,486 A1 beschrieben.

Es ist bekannt, dass es bei der destillativen Aufarbeitung des Reaktoraustrags aus der homogen durchgeführten rhodiumkatalysierten Hydroformylierungsreaktion zu einer Zersetzung und/oder Desaktivierung des Rhodiumkatalysators kommen kann. Bei diesen Vorgängen kann es zum einen zur Abscheidung von unlöslichen Rhodiumverbindungen oder Rhodiummetall kommen, die sich in den Apparaturen als Niederschläge oder Beläge festsetzen und nicht mehr in den homogen durchgeführten Hydroformylierungsprozess rezirkuliert werden können. Solche Rhodiummengen stehen für den laufenden Prozess zunächst einmal nicht mehr zur Verfügung und können nur während eines Anlagenstillstands durch gezielte Reinigungsmaßnahmen aus den Apparaturen zurückgewonnen werden. Des Weiteren können durch Zersetzung und Desaktivierung anfallende rhodiumhaltige Feststoffe in dem Destillationsrückstand zwar in suspendierte und damit pumpfähiger Form vorliegen und in den Hydroformylierungsreaktor zurückgeführt werden, doch zeigen solche suspendierte rhodiumhaltige Feststoffe häufig nur noch eine geringe Hydroformylierungsaktivität. Das kann ebenso für Zersetzungs- und Desaktivierungsprodukte gelten, die in dem Destillationsrückstand in gelöster Form vorliegen. Zur Aufrechterhaltung einer vorgegebenen Raum-Zeit-Ausbeute an Aldehyd muss daher mehr Rhodium in Form eines frischen Rhodiumkatalysators zudosiert werden als Rhodium mit den gebildeten hochsiedenden Aldehydkondensationsprodukten entnommen wird. Die Differenz zwischen der zugesetzten Menge an frischem Rhodium und der ausgeschleusten Menge an desaktiviertem Rhodium verbleibt in der Anlage und gilt zunächst als Rhodiumverbrauch.

Der auf den Einsatz der olefinisch ungesättigten Verbindung bezogene Rhodiumverbrauch oder der spezifische Rhodiumverbrauch beeinflusst in erheblichem Maße die Wirtschaftlichkeit der zweiten, homogen durchgeführten Hydroformylierungsstufe des aus EP 0 805 138 A1 bekannten Prozesses, da das Edelmetall Rhodium sehr teuer ist. Auch erschweren die erheblichen Preisschwankungen für Rhodium die Wirtschaftlichkeitsplanung.

Zur Verminderung der Katalysatordesaktivierung während der Aufarbeitung des Reaktoraustrags bei der rhodiumkatalysierten, homogen durchgeführten Hydroformylierungsreaktion von olefinisch ungesättigten Verbindungen sind im Stand der Technik mehrere Verfahren bekannt.

Bei der aus DE 102 20 801 A1 bekannten Arbeitsweise wird der Hydroformylierungsaustrag nur so weit aufkonzentriert, dass die Rhodiumkonzentration in der in die Hydroformylierungszone zurückgeführten Lösung 20 bis 150 Massen-ppm beträgt. Von einer höheren Aufkonzentrierung wird abgeraten, um nicht den Rhodiumkatalysator thermisch zu stark zu belasten und seine Zersetzung und Desaktivierung zu fördern.

Nach dem Verfahren nach DE 100 48 301 A1 erfolgt die Stabilisierung des Rhodiumkatalysators während der Aufarbeitung des rohen Hydroformylierungsprodukts durch die Beaufschlagung mit Kohlenmonoxid. Dazu wird der Hydroformylierungsaustrag zunächst entspannt und die dabei anfallende Flüssigkeit in eine Kopffraktion und in eine Sumpffraktion aufgetrennt. Die anfallende Sumpffraktion wird unter die Temperatur des Austrags des Hydroformylierungsreaktors abgekühlt und mit Kohlenmonoxid beaufschlagt. Die erhaltene Rhodium enthaltende Lösung kann so gelagert werden und in den Hydroformylierungsprozess zurückgeführt werden.

Ebenfalls ist bekannt, dem Reaktionsaustrag aus der Hydroformylierungsreaktion vor der destillativen Aufarbeitung einen Komplexbildner, beispielsweise Triphenylphosphin, zur Stabilisierung zuzusetzen. Nach dem aus EP 0 272 608 A1 bekannten Verfahren wird der Hydroformylierungsschritt in Gegenwart eines Rhodiumkatalysators und Triphenylphosphinoxid durchgeführt und der Hydroformlierungsaustrag vor der destillativen Aufarbeitung mit Triphenylphosphin zur Stabilisierung versetzt. Nach Abtrennung der flüchtigen organischen Bestandteile, wie Aldehyde und Alkohole, wird der rhodiumhaltige Rückstand oxidiert und Triphenylphosphin in Triphenylphosphinoxid überführt. Anschließend wird der oxidierte Katalysatorrückstand wieder in die Hydroformylierungszone zurückgeführt.

WO 01/58844 A2 behandelt ebenfalls die Aufarbeitung eines flüssigen Austrags einer kontinuierlich durchgeführten Hydroformylierungsreaktion. Bei dem bekannten Verfahren erfolgt eine zweistufige Entspannung, wobei in der ersten Stufe auf einen Druck entspannt wird, der 0,2 bis 2 MPa unterhalb des Reaktordruckes liegt. Die dabei anfallende flüssige Phase wird in einer zweiten, niedrigeren Druckstufe weiter entspannt, wobei sich eine Gasphase bildet, die im Wesentlichen die Hauptmenge des gewünschten Hydroformylierungsprodukts enthält. Die dabei anfallende Flüssigphase, die hochsiedende Nebenprodukte und den homogen gelösten Hydroformylierungskatalysator enthält, wird entweder direkt oder nach destillativer Aufarbeitung in den Hydroformylierungsreaktor zurückgeführt. Im Falle einer destillativen Aufarbeitung wird die Flüssigphase aus der zweiten Entspannungsstufe auf den Kopfteil und die Gasphase aus der zweiten Entspannungsstufe auf den Boden einer Destillationskolonne gegeben. Durch diese Gegenstromfahrweise kommt es zu einem intensiven Stoffaustausch. Um Katalysatorschädigungen zu vermeiden, arbeitet man in der Destillation bei einem möglichst niedrigen Druck. Der katalysatorhaltige Sumpfaustrag aus der Destillationskolonne wird anschließend in den Hydroformylierungsreaktor zurückgeführt.

Es bestand somit die Aufgabe, ein Verfahren bereitzustellen, das es unter ökonomisch vertretbaren Bedingungen erlaubt, olefinisch ungesättigte Verbindungen, die im Abgas einer mit wässriger Katalysatorlösung durchgeführten Hydroformylierungsreaktion enthalten sind, zu Carbonylverbindungen umzusetzen, und das sich durch einen geringen Katalysatorverbrauch auszeichnet.

Die Erfindung besteht daher in einem Verfahren zur Hydroformylierung olefinisch ungesättigter Verbindungen, wobei die Reaktion in einer ersten Reaktionsstufe in einem heterogenen Reaktionssystem unter Verwendung einer wässrigen Lösung, wasserlösliche organische Phosphor(III)-Verbindungen in komplexer Bindung enthaltender Rhodiumverbindungen als Katalysatoren bei Drücken von 0,4 bis 10 MPa erfolgt und Abgas gebildet wird, und wobei das Abgas der ersten Reaktionsstufe einer zweiten Reaktionsstufe zugeführt wird, in der im Abgas noch vorhandene Restmengen der olefinisch ungesättigten Verbindungen in einem homogenen Reaktionssystem in Gegenwart von Komplexverbindungen des Rhodiums mit organischen Phosphor(III)-Verbindungen als Katalysatoren bei Drücken von 15 bis 40 MPa umgesetzt werden. Es ist dadurch gekennzeichnet, dass man
a) den Austrag der zweiten Reaktionsstufe in einen Entgasungsbehälter bei gleichem oder geringerem Druck leitet, wobei eine Auftrennung in eine Flüssigphase und in eine Gasphase, die als Abgas abgeführt wird, erfolgt; und
b) die in dem Entgasungsbehälter erhaltene Flüssigphase in einer ersten Entspannungsstufe auf einen Druck entspannt, der niedriger als der Druck in dem Entgasungsbehälter ist, wobei eine Auftrennung in eine Flüssigphase und in eine Gasphase erfolgt und wobei die Gasphase unter Bildung eines Abgases kondensiert wird, das abgeführt wird und in dem der Kohlenmonoxidgehalt mehr als 10 Volumenprozent beträgt; und
c) die in der ersten Entspannungsstufe erhaltene Flüssigphase in einer zweiten Entspannungsstufe auf einen Druck entspannt, der niedriger als der Druck der ersten Entspannungsstufe ist, wobei eine Auftrennung in eine Flüssigphase und in eine Gasphase erfolgt und wobei die Gasphase unter Bildung eines Abgases kondensiert wird, das abgeführt wird; und
d) die in der zweiten Entspannungsstufe erhaltene Flüssigphase auf eine Destillationskolonne gibt, wobei eine Auftrennung erfolgt in eine Flüssigphase, die im Wesentlichen hochsiedende Nebenprodukte der Hydroformylierung, den homogen gelösten Rhodiumkatalysator und geringe Mengen an Hydroformylierungsprodukt enthält, und in eine Gasphase, die das Hydroformylierungsprodukt enthält, und
e) aus der Destillationskolonne bei einer Sumpftemperatur von höchstens 150°C einen flüssigen rhodiumhaltigen Austrag abführt, der teilweise ausgeschleust und teilweise in die zweite Reaktionsstufe zurückgeführt wird, und
f) dem in die zweite Reaktionsstufe zurückgeführten flüssigen rhodiumhaltigen Austrag frisches Rhodium in einer solchen Menge zusetzt, dass das Massenverhältnis von zugesetztem frischen Rhodium zu ausgeschleustem Rhodium von 3 :1 bis 1 : 3, bevorzugt 1:1, beträgt.

Die erste Reaktionsstufe des neuen Verfahrens führt man als heterogene Reaktion in einem Zweiphasensystem durch, eine Umsetzung, die zum Beispiel in der DE-B-26 27 354 beschrieben ist. Dieser Prozess ist charakterisiert durch das Vorliegen einer organischen Phase, die das olefinische Ausgangsmaterial und das Reaktionsprodukt enthält und einer wässrigen Phase, in der der Katalysator gelöst ist. Als Katalysatoren werden wasserlösliche Rhodium-Komplexverbindungen eingesetzt, die wasserlösliche organische Phosphor(III)-Verbindungen als Liganden enthalten. Beispiele für wasserlösliche Phosphor(III)-Verbindungen, die mit Rhodium Komplexverbindungen bilden, sind Triarylphosphine, Trialkylphosphine und aromatische oder gemischt aliphatisch-aromatische Bisphosphine, deren organische Reste Sulfonsäuregruppen oder Carboxylgruppen enthalten. Ihre Herstellung und Anwendung ist zum Beispiel aus DE-B 26 27 354, EP 0 103 810 B1, EP 0 163 234 B1 und EP 0 571 819 A1 bekannt. Weitere Gruppen geeigneter Verbindungen sind sulfonierte oder carboxylierte organische Bisphosphite sowie heterocyclische Verbindungen des dreibindigen Phosphors (zum Beispiel EP 0 575 785 A1, EP 0 646 588 A1).

Die Bedingungen, unter denen die Umsetzung in der ersten Reaktionsstufe abläuft, können innerhalb weiter Grenzen variiert und den individuellen Gegebenheiten angepasst werden. Sie hängen unter anderem vom Einsatzmaterial, vom ausgewählten Katalysatorsystem und vom angestrebten Umsetzungsgrad ab. Üblicherweise führt man die Hydroformylierung der Einsatzstoffe bei Temperaturen von 50 bis 180°C durch. Bevorzugt hält man Temperaturen von 90 bis 150°C und insbesondere von 110 bis 140°C ein. Der Gesamtdruck erstreckt sich über einen Bereich von 0,4 bis 10 MPa, vorzugsweise 1 bis 6 MPa und insbesondere 1,5 bis 5 MPa. Das molare Verhältnis von Wasserstoff zu Kohlenmonoxid bewegt sich üblicherweise zwischen 1 : 10 bis 10 : 1, Mischungen, die Wasserstoff und Kohlenmonoxid im Verhältnis 3 : 1 bis 1 : 3 und insbesondere etwa 1 : 1 enthalten, sind besonders geeignet.

Die Rhodium-Konzentration beträgt 20 bis 1000 Gew.-ppm, vorzugsweise 50 bis 500 Gew.-ppm und insbesondere 100 bis 300 Gew.-ppm, jeweils bezogen auf die wässrige Katalysatorlösung. Obgleich es möglich ist, als Katalysator die stöchiometrisch zusammengesetzte Rhodium-Phosphor-Komplexverbindung einzusetzen, arbeitet man üblicherweise in Gegenwart von überschüssigem Phosphorliganden, d.h. Ligand, der mit Rhodium keine komplexe Bindung eingegangen ist. Je mol Rhodium wendet man bevorzugt 3 bis 200 mol Phosphor(III)-Verbindungen in Form einer wasserlöslichen organischen Phosphorverbindung an. Besonders bewährt haben sich molare Verhältnisse von Rhodium zu Phosphor im Bereich von 1 : 50 bis 1 : 100. Der Rhodium-Phosphor-Komplexkatalysator braucht nicht einheitlich zusammengesetzt sein, sondern kann zum Beispiel aus einem Gemisch von Rhodium-Komplexverbindungen bestehen, die sich durch die Art der Phosphorliganden unterscheiden. Ebenso kann der in der wässrigen Katalysatorlösung enthaltene freie Phosphorligand aus einem Gemisch unterschiedlicher wasserlöslicher organischer Phosphor(III)-Verbindungen zusammengesetzt sein. Man bildet den Katalysator üblicherweise aus den Komponenten Rhodium oder Rhodiumverbindung, wasserlösliche organische Phosphor(III)-Verbindung und Synthesegas unter den Bedingungen der Hydroformylierungsreaktion im Reaktionsgemisch. Er kann der Reaktionsstufe aber auch präformiert, das heißt gesondert hergestellt, zugeführt werden.

Auch hinsichtlich der verfahrenstechnischen und apparativen Ausgestaltung der ersten Stufe des neuen Verfahrens kann man sich innerhalb weiter Grenzen bewegen. Eine bewährte Ausführungsform der heterogenen Hydroformylierung unter Verwendung einer wässrigen Katalysatorphase ist in der EP 0 103 810 B1 beschrieben. Das Reaktionsprodukt wird in einem Phasentrenner von der wässrigen Katalysatorlösung, die in den Prozess zweckmäßigerweise zurückgeführt wird, getrennt. Da die Phasentrennung unter sehr schonenden Bedingungen erfolgt und Rhodium nahezu vollständig gelöst in der wässrigen Phase vorliegt, treten in dieser Prozessstufe nur sehr geringe Rhodiumverluste auf. Eine im Laufe des technischen Betriebs nachlassende Katalysatoraktivität gleicht man durch Zufuhr von einer wässrigen Lösung mit Frischkatalysator oder durch eine Erhöhung der Reaktortemperatur aus.

Üblicherweise strebt man in der ersten Reaktionsstufe einen möglichst weitgehenden Umsatz der olefinisch ungesättigten Verbindungen an.

In Einzelfällen kann jedoch auch auf einen mehr oder minder großen Teilumsatz hin gearbeitet werden.

Das aus der ersten Reaktionsstufe entweichende Abgas, der Abgasstom, setzt sich zusammen aus dem Abgas, das dem Reaktor unmittelbar entnommen wird, dem Reaktorabgas, um eine Anreicherung von Inerten in dem, im Kreis geführten Gasgemisch zu vermeiden, und den gasförmigen Anteilen, die bei der Trennung von Katalysatorlösung und rohem Reaktionsprodukt im Phasentrenner auftreten, dem Produktabgas. Der der ersten Reaktionsstufe entnommene Abgasstrom, also Reaktorabgas und Produktabgas zusammen, besteht im Wesentlichen aus nicht umgesetzter olefinisch ungesättigter Verbindung, Kohlenmonoxid, Kohlendioxid, Wasserstoff und den Hydrierungsprodukten der olefinisch ungesättigten Verbindung. Dieses Gasgemisch wird anschließend kondensiert, wobei ein flüssiges Abgaskondensat und ein Abgas anfällt. Das Abgas wird einem Kompressor zugeführt und dann gemeinsam mit frischem Synthesegas, also einem Gemisch aus Kohlenmonoxid und Wasserstoff, und gegebenenfalls Wasserstoff der zweiten Reaktionsstufe zugeführt. Das flüssige Abgaskondensat wird direkt ohne weitere Zwischenbehandlung oder nach dem Durchleiten durch Filtereinrichtungen, die zur Abtrennung von Wasser und in Wasser vorhandener Stoffe, beispielsweise gelöster Komplexliganden dienen, ebenfalls als Einsatzmaterial in die zweite Reaktionsstufe eingespeist.

Die zweite Reaktionsstufe wird unabhängig von der ersten betrieben. In ihr werden die im Abgasstrom vorhandenen Restmengen der olefinisch ungesättigten Verbindungen in einem homogenen Reaktionssystem mit Kohlenmonoxid und Wasserstoff umgesetzt. Der Begriff homogenes Reaktionssystem steht für eine im Wesentlichen aus Lösungsmittel, Katalysator, olefinisch ungesättigter Verbindung und Reaktionsprodukt zusammengesetzte homogene Lösung. Als Katalysatoren werden Rhodium-Komplexverbindungen verwendet, die organische Phosphor(III)-Verbindungen als Liganden enthalten. Derartige Komplexverbindungen und ihre Herstellung sind bekannt (zum Beispiel US 3 527 809 A1, US 4 148 830 A1, US 4 247 486 A1, US 4 283 562 A1). Sie können als einheitliche Komplexverbindung oder auch als Gemisch unterschiedlicher Komplexverbindungen eingesetzt werden. Die Rhodium-Konzentration im Reaktionsmedium erstreckt sich über einen Bereich von etwa 1 bis etwa 1000 Gew.-ppm und beträgt vorzugsweise 10 bis 500 Gew.-ppm. Insbesondere wendet man Rhodium in Konzentrationen von 10 bis 200 Gew.-ppm, jeweils bezogen auf die eingesetzte olefinisch ungesättigte Verbindung. Wie in der ersten Stufe kann als Katalysator die stöchiometrisch zusammengesetzte Rhodium-Komplexverbindung Anwendung finden. Es hat sich jedoch als zweckmäßig erwiesen, die Hydroformylierung in Gegenwart eines Katalysatorsystems aus Rhodium-Phosphor-Komplexverbindung und freiem, d.h. überschüssigem Phosphorliganden durchzuführen, der mit Rhodium keine Komplexverbindung mehr eingeht. Der freie Phosphorligand kann der gleiche sein wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Phosphor-Komplexverbindungen, die als Katalysatoren Anwendung finden können, sind in US 3 527 809 A1 beschrieben. Zu den bevorzugten Liganden in den Rhodium-Komplexkatalysatoren zählen zum Beispiel Triarylphosphine wie Triphenylphosphin, Trialkylphosphine wie Tri(n-octyl)phosphin, Trilaurylphosphin, Tri(cyclohexyl)phosphin, Alkylphenylphosphine, Cycloalkylphenylphosphine und organische Bisphosphite. Wegen seiner leichten Zugänglichkeit wird Triphenylphosphin besonders häufig angewandt.

Üblicherweise beträgt das molare Verhältnis von Rhodium zu Phosphor 1 : 1 bis 1 : 300, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 3 bis 1 : 200 ein. Bei Anwendung von Triarylphosphinen haben sich insbesondere Rh/P-Molverhältnisse von 1 : 30 bis 1 : 150 bewährt.

Die Hydroformylierungsreaktion wird in Gegenwart eines Lösungsmittels ausgeführt. Als Lösungsmittel werden organische Verbindungen eingesetzt, in denen Ausgangsmaterial, Reaktionsprodukt und Katalysatorsystem löslich sind. Beispiele für solche Verbindungen sind aromatische Kohlenwasserstoffe wie Benzol, Toluol oder die Xylole. Andere gebräuchliche Lösungsmittel sind Paraffinöl, Ketone oder Ether. Als besonders geeignete Lösungsmittel haben sich die hochsiedenden Kondensationsverbindungen der Aldehyde erwiesen, die als hochsiedende Nebenprodukte bei der Hydroformylierung entstehen. Der Anteil des Lösungsmittels im Reaktionsmedium kann über einen weiten Konzentrationsbereich variiert werden und beträgt üblicherweise zwischen 20 und 90 Gew.-%, vorzugsweise 50 bis 80 Gew.-%, bezogen auf das Reaktionsgemisch.

Der Reaktionsdruck in der zweiten Stufe des Gesamtprozesses liegt im Bereich von 15 bis 40 MPa. Besonders bewährt hat es sich, Drücke zwischen 15 und 35 MPa, vorzugsweise 20 bis 30 MPa einzuhalten. Derartige Bereiche sind für Hydroformylierungen in homogenen Reaktionssystemen und in Gegenwart von Komplexverbindungen des Rhodiums mit organischen Phosphor(III)-Verbindungen ungewöhnlich, unabhängig davon, ob die Umsetzung ein- oder mehrstufig erfolgt. Das Volumenverhältnis von Wasserstoff zu Kohlenmonoxid beträgt 1 : 10 bis 10 : 1, vorzugsweise 1 : 3 bis 3 : 1 und insbesondere 1 : 1.

Die Reaktionstemperaturen betragen in der zweiten Stufe des neuen Prozesses 50 bis 160°C. Temperaturen von 60 bis 150°C und insbesondere 75 bis 140°C werden bevorzugt.

Die zweite Reaktionsstufe kann mit einem oder mehreren parallel oder hintereinander betriebenen Hydroformylierungsreaktoren ausgestaltet werden. Durch die zweite Reaktionsstufe wird sichergestellt, dass der größte Teil der im Abgas enthaltenen, in der ersten Stufe nicht umgesetzten olefinisch ungesättigten Verbindungen hydroformyliert wird. Obwohl die olefinisch ungesättigten Verbindungen im Abgas in beträchtlicher Verdünnung vorliegen, kann die zweite, unter Hochdruck homogen durchgeführte Reaktionsstufe mit hoher Effizienz durchgeführt werden. So beträgt zum Beispiel bei der Hydroformylierung niedriger Olefine der Anteil des nicht umgesetzten olefinischen Einsatzmaterials im Abgas nur etwa 20 bis 70 Gew.-%. Derartige Konzentrationen stehen bei dieser Reaktionsart einer weitgehenden Umsetzung der ungesättigten Ausgangsverbindung entgegen. Die mit dem Abgas aus der ersten Reaktionsstufe ausgetragenen Verunreinigungen, beispielsweise Wasser, dem Lösungsmittel für den Katalysator aus der ersten Reaktionsstufe, oder schwefelhaltige Abbauprodukte des Katalysatorsystems, wie Mercaptane, können in gewissen Grenzen zugelassen werden, obwohl Mercaptane in dem organischen Reaktionsmedium gut löslich sind und bekanntermaßen als Katalysatorgifte wirken können. Auch Wasser kann den organischen Phosphorliganden aus der homogen gelösten Rhodiumkomplexverbindung unter Bildung katalytisch inaktiver Substanzen verdrängen. Auch können im Wasser gelöste Verbindungen, beispielsweise mitgerissene Mengen der in der ersten Reaktionsstufe verwendeten wasserlöslichen organischen Phosphor(III)-Verbindungen, den homogen gelösten Rhodiumkomplex schädigen. Es empfiehlt sich daher, zwischen der ersten heterogen durchgeführten Hydroformylierungsstufe und der nachfolgenden homogen durchgeführten Hydroformylierungsstufe eine Trenneinrichtung, beispielsweise eine Filtereinrichtung, zu installieren, um im Abgaskondensat der ersten Hydroformylierungsstufe vorhandene Verunreinigungen abzutrennen und ihren Eintrag in die zweite Reaktionsstufe zu verhindern. Eine derartige Prozessführung wird in der Patentanmeldung (AKZ: 10 2009 004 655.0) beschrieben.

Der flüssige Austrag der zweiten Reaktionsstufe wird anschließend über Druckentspannungsstufen und Destillationseinrichtungen aufgearbeitet. Zunächst wird der flüssige Austrag in einen Entgasungsbehälter geleitet, in dem eine Auftrennung in eine Flüssigphase und in eine Gasphase erfolgt. Den Entgasungsbehälter, in dem die Entgasungsstufe durchgeführt wird, bezeichnet man auch als Hochdruckabscheider. Die dabei anfallende Gasphase enthält im Wesentlichen überschüssiges Synthesegas, nicht umgesetzte olefinisch ungesättige Verbindung, hydrierte Produkte und Inertgase aus dem Herstellprozess für Synthesegas. Die Gasphase wird als Abgas, sogenanntes Hochdruckabgas, abgeführt und thermisch verwertet. Die erhaltene Flüssigphase enthält im Wesentlichen das Hydroformylierungsprodukt, hochsiedende Nebenprodukte der Hydroformylierung, wie hochsiedende Aldehydkondensationsprodukte, Rhodiumkomplexverbindungen sowie gelöstes Synthesegas. Falls in der zweiten Reaktionsstufe verwendet, ist noch ein weiteres organisches Lösungsmittel, wie Toluol oder Xylol zugegen. Vorzugsweise verwendet man jedoch die hochsiedenden Aldehydkondensationsprodukte als Lösungsmittel ohne Zusatz eines weiteren organischen Lösungsmittels. Die Entgasungsstufe dient zur Entfernung von in dem flüssigen Austrag gelösten gasförmigen Anteilen und wird daher bei einem Druck durchgeführt, der gleich dem Druck in der zweiten Reaktionsstufe ist oder geringer ist. Dabei liegt der Druck in der Entgasungsstufe nur geringfügig unter dem Reaktordruck und entspricht im Allgemeinen dem Druckverlust zwischen dem Reaktor in der zweiten Reaktionsstufe und dem nachgeschalteten Entgasungsbehälter.

Die im Hochdruckabscheider abgeschiedene Flüssigphase wird anschließend als flüssiger Strom aus dem Entgasungsbehälter ausgetragen und in einer ersten Entspannungsstufe in einem sogenannten Mitteldruckabscheider auf einen Druck entspannt, der niedriger als der Druck in der Entgasungsstufe ist. Vorzugsweise wird in dieser ersten Entspannungsstufe auf einen Druck entspannt, der bei 0,1 bis 3,0 MPa, insbesondere 0,5 bis 2,0 MPa, liegt. Auch in dieser ersten Entspannungsstufe erfolgt eine Auftrennung in eine Gasphase und Flüssigphase. Neben Synthesegas enthält die Gasphase aus der ersten Entspannungsstufe im Wesentlichen noch die unumgesetzte olefinisch ungesättigte Verbindung und Hydrierprodukte. Die Gasphase wird kondensiert, Kondensate werden als Wertprodukte in den Prozess zurückgeführt und das nicht kondensierte Abgas wird abgeführt und einer thermischen Nutzung zugeführt. Wesentlich für das Gelingen des erfindungsgemäßen Verfahrens ist, in dem abgeführten Abgas der ersten Entspannungsstufe einen Kohlenmonoxidgehalt von mehr als 10 Volumenprozent einzustellen. Vorzugsweise liegt der Kohlenmonoxidgehalt in einem Bereich von 15 bis 40, insbesondere von 18 bis 30 Volumenprozent. Für die Stabilisierung der Rhodiumkomplexverbindung und für die Vermeidung der Katalysatordesaktivierung und der Abscheidung von Feststoffen, wie von metallischem Rhodium, ist daher an dieser Stelle des Verfahrens das Einhalten einer bestimmten Kohlenmonoxidkonzentration erforderlich. Die Kohlenmonoxidkonzentration in dem abgeführten Abgas des Mitteldruckabscheiders kann beispielsweise dadurch justiert werden, dass aus dem Hochdruckabscheider ein entsprechend hoher Abgasstrom und damit ein hoher Inerteanteil aus dem Prozess ausgeschleust wird. Das aus dem Mitteldruckabscheider abgeführte Abgas, das die kritische Kohlenmonoxidkonzentration aufzuweisen hat, bezeichnet man auch als Mitteldruckabgas. Die Messung des Kohlenmonoxidgehaltes im abgeführten Abgas erfolgt durch Abgasanalyse nach üblichen Methoden.

Die in der ersten Entspannungsstufe erhaltene Flüssigphase enthält im Wesentlichen das gewünschte Hydroformylierungsprodukt, Rhodiumkomplexverbindungen, hochsiedende Aldehydkondensationsprodukte als Lösungsmittel und gegebenenfalls ein weiteres organisches Lösungsmittel. Es wird anschließend in einer zweiten Entspannungsstufe in einem Niederdruckabscheider auf einen Druck entspannt, der niedriger als der Druck der ersten Entspannungsstufe ist, wobei wiederum eine Auftrennung in eine Flüssigphase und in eine Gasphase erfolgt. Vorzugsweise wird in dieser zweiten Entspannungsstufe auf einen Druck entspannt, der bei 0,1 bis 0,5 MPa, insbesondere bei 0,1 bis 0,3 MPa, liegt. Die dabei anfallende Gasphase enthält überwiegend leichtsiedende Komponenten, wie die unumgesetzte olefinisch ungesättigte Verbindung oder Hydrierprodukte sowie geringe Mengen an Synthesegas und geringe Mengen an dem gewünschten Hydroformylierungsprodukt. Die Gasphase wird kondensiert, Kondensate werden in den Prozess zurückgeführt und nicht kondensiertes Abgas wird als Niederdruckgas abgeführt und einer thermischen Nutzung zugeführt.

Die im Niederdruckabscheider erhaltene Flüssigphase wird zur Abtrennung des gewünschten Hydroformylierungsprodukts vom Rhodiumkatalysator auf eine Destillationskolonne gegeben, vorzugsweise auf den Mittelteil. Es kann eine übliche Destillationskolonne verwendet werden, die zur Verbesserung der Trennleistung mit Füllkörpern, wie Raschig-Ringen, Spiralen oder Sattelkörpern, Packungen oder Einbauten, wie Rieselböden ausgestattet sein kann. Die Destillationsbedingungen, wie Temperatur und Druck, richten sich nach den physikalischen Eigenschaften der gewünschten Aldehyde. Um die Desaktivierung des gelösten Rhodiumkomplexkatalysators und die Abscheidung von Rhodiumverbindungen oder Rhodiummetall zu vermeiden, wird der flüssige Austrag aus der Destillationskolonne bei einer Sumpftemperatur von höchstens 150°C, vorzugsweise zwischen 100 und 150°C, abgeführt. Die Destillationskolonne wird im Allgemeinen bei Normaldruck betrieben, obwohl auch höhere oder niedrigere Drücke, je nach abzutrennenden Aldehyden, eingestellt werden können. Wird beispielsweise Propylen als olefinisch ungesättigte Verbindung verarbeitet, wird der gewünschte Butyraldehyd bei einer Sumpftemperatur zwischen 110 und 130°C unter Normaldruck destilliert. Das Kopfprodukt der Destillationskolonne enthält das gewünschte Hydroformylierungsprodukt, das zur Auftrennung in den n- und iso-Aldehyd auf eine weitere Kolonne geben wird.

Der über den Sumpf der Destillationskolonne entnommene Flüssigkeitsstrom, der im Wesentlichen aus hochsiedenden Aldehydkondensationsprodukten und organischem Lösungsmittel, falls zugesetzt, besteht, in dem der Rhodiumkatalysator homogen gelöst vorliegt und der weitere Rhodiumverbindungen von geringerer katalytischer Aktivität teils in suspendierte Form enthält, wird teilweise ausgeschleust, um ein Aufkonzentrieren der Aldehydkondensationsprodukte zu vermeiden. Im stationären Betriebszustand der Hydroformylierungsanlage bleibt die Konzentration an Aldehydkondensationsprodukten konstant, da die während der Hydroformylierungsreaktion gebildete Menge in etwa der ausgeschleusten Menge entspricht.

Der nicht ausgeschleuste Teil wird in die zweite Reaktionsstufe zurückgeführt. Dem zurückgeführten flüssigen Strom werden frisches Rhodium und freie organische Phosphor(III)-Verbindung, vorzugsweise die bereits im katalytischen Prozess eingesetzte Phosphorverbindung, zugesetzt. Man setzt die organische Phosphor(III)-Verbindung in einer solchen Menge zu, dass das molare Verhältnis von Rhodium zu Phosphor 1 : 3 bis 1 : 200, vorzugsweise 1 : 30 bis 1 : 150 beträgt.

Frisches Rhodium wird in Form von Rhodiumverbindungen, wie Rhodiumsalzen, beispielsweise Salze aliphatischer Mono- und Polycarbonsäuren, wie Rhodium-2-ethylhexanoat, -acetat, -oxalat, -propionat oder -malonat, Salze anorganischer Wasserstoff- und Sauerstoffsäuren, wie Nitrat, Sulfat oder Chlorid, Rhodiumkomplexverbindungen wie Cyclopentadienylrhodiumverbinzungen, Rhodiumacetylacetonat, [RhCl(Cyclooctadien-1,5)]₂, Rhodiumcarbonylverbindungen wie Rh₃(CO)₁₂, Rh₆(CO)₁₆ oder in Form der verschiedenen Rhodiumoxide in einer solchen Menge zugesetzt, dass das Massenverhältnis von zugesetztem frischem Rhodium zu über die Aldehydkondensationsprodukte ausgeschleustem Rhodium von 3 : 1 bis 1 : 3 beträgt, bevorzugt 1 : 1. Bevorzugt wird Rhodium in Form von Rhodium-2-ethylhexanoat zugesetzt.

Die für den frischen Rhodiumzusatz verwendete Rhodiumverbindung wird in einem organischen Lösungsmittel gelöst oder, je nach Lösungsverhalten, in dem organischen Lösungsmittel suspendiert. Als zweckmäßige Lösungsmittel haben sich die hochsiedenden Aldehydkondensationsprodukte oder bevorzugt die gewünschten Aldehyde erwiesen.

Nach Zusatz von freier organischer Phosphor(III)-Verbindung und frischem Rhodium, zweckmäßigerweise in Form von Rhodium-2-ethylhexanoat, zu der zurückgeführten, rhodiumhaltigen Lösung wird die so eingestellte Lösung wieder in die zweite, unter Hochdruck durchgeführte Reaktionsstufe gegeben.

Neben dem Einstellen des Kohlenmonoxidgehalts in dem abgeführten Abgas des Mitteldruckabscheiders (Mitteldruckabgas) auf einen Wert von mehr als 10 Volumenprozent, vorzugsweise von 15 bis 40 Volumenprozent, kommt dem Massenverhältnis von ausgeschleustem Rhodium zu frisch zugesetztem Rhodium für die Reduzierung des spezifischen Rhodiumverbrauchs eine entscheidende Bedeutung zu. Wird zuviel Rhodium über die hochsiedenden Aldehydkondensationsprodukte ausgeschleust, muss zuviel frisches Rhodium zugesetzt werden, um pro Zeiteinheit und Reaktorvolumeneinheit eine ausreichende Menge an Hydroformylierungsprodukten zu erhalten. Wird hingegen zuviel Rhodium zurückgeführt, beobachtet man überraschenderweise auch einen Abfall in der Raum-Zeit-Ausbeute.

Ohne zu sehr auf mechanistische Überlegungen eingehen zu wollen, kann man annehmen, dass in dem zurückgeführten Flüssigkeitsstrom Rhodium in Form von solchen Verbindungen vorliegt, die sich unter den Hochdruckhydroformylierungsbedingungen und in den nachfolgenden Entspannungs- und Destillationsschritten bevorzugt als rhodiumhaltige Feststoffe in den Apparaten abscheiden. Derartige Feststoffe scheinen keine oder nur noch eine geringe Hydroformylierungsaktivität zu besitzen. Da sie in den Apparaten zum Teil verbleiben und mit dem Flüssigkeitsstrom nicht ausgetragen werden, muss vermehrt frisches Rhodium zugesetzt werden, um die gewünschte Raum-Zeit-Ausbeute zu halten, obwohl eine vergleichsweise hohe Rhodiummenge über den Sumpf der Destillationskolonne in die zweite Reaktionszone zurückgeführt wird. Auch kann vermutet werden, dass die zurückgeführten Rhodiumverbindungen als Kristallisationskeime wirken und die Umwandlung der katalytisch aktiven Rhodiumverbindungen in inaktive Feststoffe beschleunigen. Erst wenn der Kohlenmonoxidgehalt in dem abgeführten Abgas des Mitteldruckabscheiders oberhalb von 10 Volumenprozent, vorzugsweise im Bereich von 15 bis 40 Volumenprozent, liegt und damit Kohlenmonoxid in einer ausreichenden Menge für die Stabilisierung der Rhodiumverbindungen zur Verfügung steht und das Massenverhältnis von zugesetztem frischem Rhodium zu über die hochsiedenden Aldehydkondensationsprodukte ausgeschleustem Rhodium von 3 :1 bis 1 : 3, vorzugsweise 1:1, beträgt, lässt sich die Abscheidung von rhodiumhaltigen Feststoffen in dem Hochdruckhydroformylierungsreaktor und in den nachgeschalteten Aufarbeitungsvorrichtungen wirksam unterdrücken.

Da die hochsiedenden Aldehydkondensationsprodukte als Lösungsmittel dienen und ihr Anteil im homogenen Reaktionsgemisch über einen vergleichsweise breiten Bereich zugelassen werden kann, kann die ausgeschleuste Rhodiummenge gezielt in Bezug auf den Frischrhodiumeinsatz eingestellt werden. Mit der so eingestellten und ausgeschleusten Rhodiummenge werden dann ebenfalls genügend hochsiedende Aldehydkondensationsprodukte aus dem Prozess entfernt und ihre Aufkonzentrierung in dem homogenen Reaktionsgemisch wird vermieden.

Die zurückgeführten Rhodiumverbindungen liegen hingegen in einer schwach aktiven Form vor. Um eine vorgegebene Raum-Zeit-Ausbeute in der Hydroformylierungsreaktion zu erzielen und um die Zugabe an frischem Rhodium möglichst geringzuhalten, muss eine vergleichsweise hohe Menge des rhodiumhaltigen Sumpfablaufs aus der Destillationskolonne in den Prozess zurückgeführt und im Kreis gefahren werden. Erst bei dem definierten Verhältnis von frisch zugesetztem Rhodium zu ausgeschleustem Rhodium von 3 : 1 bis 1 : 3, vorzugsweise 1 : 1, wird gerade eine so hohe Kreislaufmenge mit Rhodium in schwach aktiver Form gefahren und gerade soviel Rhodium ausgeschleust, dass das zurückgeführte Rhodium bei der eingestellten Kohlenmonoxidkonzentration ausreichend stabil ist und nur in einer solchen Menge vorliegt, dass frisch zugesetztes Rhodium nicht geschädigt wird. Für das Erreichen der gewünschten Raum-Zeit-Ausbeute muss daher vergleichsweise wenig frisches Rhodium im Bereich von 0,5 bis 3 Gew.-ppm, bezogen auf die olefinisch ungesättigte Verbindung, zugesetzt werden. Arbeitet man hingegen nach dem aus EP 0 805 138 A1 bekannten Verfahren, liegt die Zugabe von frischem Rhodium im Bereich von 3 bis 6 Gew.-ppm, bezogen auf die olefinisch ungesättigte Verbindung.
Daraus resultieren dann entsprechend höhere Rhodiumverbräuche in dem bekannten Verfahren.

Vorzugsweise wird der Teil des rhodiumhaltigen Sumpfaustrags aus der Destillationskolonne, der ausgeschleust werden soll, noch über eine zweite Destillationskolonne, die Nachlaufkolonne, geleitet. Die Nachlaufkolonne wird unter solchen Bedingungen betrieben, dass ein Teil der hochsiedenden Aldehydkondensationsprodukte, die noch eine gewisse Flüchtigkeit besitzen, über den Kopfbereich und aus einem Seitenabzug im unteren Kolonnenteil abgeführt wird, während ein Teil des rhodiumhaltigen Sumpfabzugs der zweiten Destillationskolonne ausgeschleust wird und der nicht ausgeschleuste Teil mit dem in die zweite Reaktionsstufe zurückgeführten flüssigen rhodiumhaltigen Austrag der Destillationskolonne vereinigt wird. Bei dieser Ausgestaltung des erfindungsgemäßen Verfahrens ergibt sich die zurückgeführte Rhodiummenge aus diesen vereinigten, rhodiumhaltigen flüssigen Strömen während die ausgeschleuste Rhodiummenge über den Sumpf der Nachlaufkolonne abgeführt wird. Die Nachlaufkolonne wird bei vergleichsweise niedrigen Temperaturen in einem Bereich von 120 bis 150°C, bevorzugt 130 bis 140°C, und bei Drücken von 5 bis 15 hPa betrieben, um die Schädigung der gelösten Rhodiumkomplexverbindungen möglichst gering zu halten. Die genaue Wahl der Destillationsbedingungen hängt von den Eigenschaften der jeweiligen Aldehydkondensationsprodukte ab. Bei der Nachlaufkolonne handelt es sich um eine übliche Kolonne, die zum Beispiel mit Füllkörpern, Packungen oder Einbauten versehen ist.

Ebenfalls hat es sich als zweckmäßig erwiesen, den rhodiumhaltigen Sumpfablauf aus der Destillationskolonne und aus der zweiten Destillationskolonne (Nachlaufkolonne) über ein Filter zu leiten, um suspendierte Feststoffe abzutrennen. Diese zum Teil rhodiumhaltigen Feststoffe erweisen sich als schädlich, und können nach ihrer Rückführung in die Hochdruckhydroformylierungszone und in die nachgeschalteten Druckabscheider und Destillationseinrichtungen den Rhodiumkatalysator schädigen und die Abscheidung weiterer rhodiumhaltiger Feststoffe fördern und so zu Rhodiumverlusten beitragen. Als Filtermedien eignen sich in der Technik übliche Materialien wie Glas- oder Metallfasern.

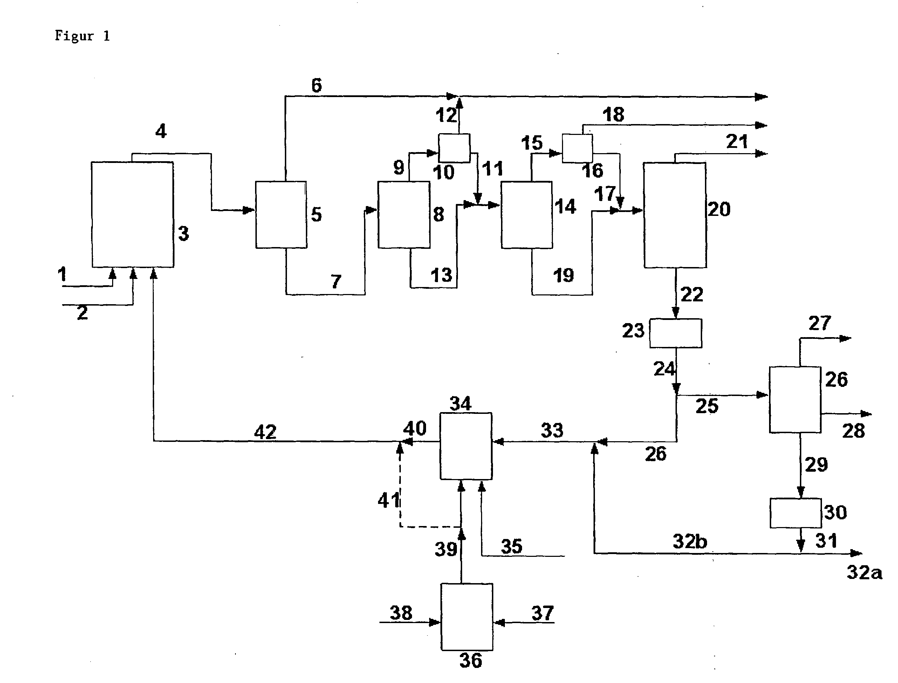

Das bevorzugte erfindungsgemäße Verfahren wird im Folgenden anhand des Prinzipschemas gemäß Figur 1 näher erläutert. Das erfindungsgemäße Verfahren ist aber nicht auf die in der Zeichnung dargestellte Ausführungsform beschränkt. Auf den Teil des Gesamtprozesses, der die erste, heterogen durchgeführte Reaktionsstufe betrifft, wird in der Figur 1 nicht eingegangen.

Das Abgaskondensat der ersten, heterogen durchgeführten Hydroformylierungsreaktion, gegebenenfalls mit olefinisch ungesättigten Verbindungen versetzt, wird über die Leitung (1) dem Hochdruckhydroformylierungsreaktor (3) zugeführt, in dem über Leitung (2) herangeführtes Synthesegas und Abgas aus der ersten Hydroformylierungsstufe eingeleitet werden. Der Hydroformylierungsaustrag wird über die Leitung (4) abgezogen und auf das Entgasungsgefäß (5), dem so genannten Hochdruckabscheider gegeben. In ihm wird das Reaktionsgemisch auf einen Druck eingestellt, der geringfügig niedriger ist als der im Hydroformylierungsreaktor (3) herrschende Druck, und in eine Gasphase, die überwiegend Synthesegas, nicht umgesetzte olefinisch ungesättigte Verbindung und leichtflüchtige Verbindungen enthält, und in eine Flüssigphase getrennt. Die Gasphase aus dem Entgasungsbehälter (5) wird über die Leitung (6) abgeführt und einer thermischen Nutzung zugeführt.

Die Flüssigphase wird über die Leitung (7) einem ersten Entspannungsgefäß (8), dem so genannten Mitteldruckabscheider, zugeführt, in dem die Flüssigkeit auf einen Druck von 1,0 bis 3,0 MPa entspannt wird. Dabei werden eine Gasphase und eine Flüssigphase gebildet. Die Gasphase enthält überwiegend Synthesegas, nicht umgesetzte olefinisch ungesättigte Verbindung und Leichtsieder und wird über die Leitung (9) abgeführt. Die Gasphase wird in einem Kondensator (10) kondensiert, beispielsweise gekühlt, und das Kondensat über Leitung (11) mit der über Leitung (13) herangeführten Flüssigphase aus dem Entspannungsgefäß (8) vereinigt. Nicht kondensiertes Abgas aus dem Kondensator (10) wird über die Leitung (12) mit dem über Leitung (6) herangeführten Abgas vereinigt und einer thermischen Nutzung zugeführt.

Wesentlich ist, dass in dem nach Kondensation über Leitung (12) abgeführten Abgas (Mitteldruckabgas) ein Kohlenmonoxidgehalt von mehr als 10 Volumenprozent, vorzugsweise im Bereich von 15 bis 40 Volumenprozent, eingestellt wird. Dies kann erreicht werden, in dem aus dem Entgasungsbehälter (5) genügend Abgas über die Leitung (6) ausgeschleust wird.

Die im Entspannungsgefäß (8) anfallende Flüssigphase wird über die Leitung (13) abgezogen, mit dem über Leitung (11) herangeführtem Kondensat vereinigt und in ein zweites Entspannungsgefäß (14), den so genannten Niederdruckabscheider gegeben, in dem die Flüssigkeit auf einen Druck von 0,1 bis 0,3 MPa entspannt wird. Dabei bildet sich eine Gasphase, die Spuren von Synthesegas, nicht umgesetzter olefinisch ungesättigter Verbindung und weitere leichtflüchtige Verbindungen, wie Hydrierprodukte, enthält und über Leitung (15) in einen Kondensator (16) geführt und dort kondensiert wird. Das flüssige Kondensat wird über die Leitung (17) mit der über Leitung (19) aus dem zweiten Entspannungsgefäß (14) herangeführten Flüssigphase vereinigt. Im Kondensator (16) angefallene, nicht kondensierte Bestandteile werden als Abgas (Niederdruckabgas) über die Leitung (18) abgeführt und einer thermischen Nutzung zugeführt.

Die erhaltene Flüssigphase enthält im Wesentlichen die Hydroformylierungsprodukte, hochsiedende Aldehydkondensationsprodukte als hochsiedende Nebenprodukte der Hydroformylierung, den rhodiumhaltigen Hydroformylierungskatalysator, weitere katalytisch weniger aktive oder inaktive Rhodiumkomplexverbindungen und gegebenenfalls Lösungsmittel. Die über Leitungen (17) und (19) herangeführten Flüssigphasen werden vereint auf die Destillationskolonne (20) gegeben, in der die gewünschten Hydroformylierungsprodukte vom Rhodium und Hochsiedern abgetrennt werden. Bei der Kolonne (20) handelt es sich um eine übliche Destillationskolonne, die zum Beispiel mit Füllkörpern, Packungen oder Einbauten für einen intensiven Gas-/Flüssigkeitsaustausch bestückt ist. Die am Kopf der Destillationskolonne (20) über die Leitung (21) abgezogenen Hydroformylierungsprodukte werden anschließend in einer weiteren Destillationsstufe in n- und iso-Aldehyd aufgetrennt (nicht in Figur 1 eingezeichnet). Der rhodiumhaltige Sumpf aus der Destillationskolonne (20) wird bei einer Sumpftemperatur von höchstens 150°C über die Leitung (22) abgeführt und auf die Filtriereinrichtung (23) gegeben, die mit einem üblichen Filtermaterial, beispielsweise aus Metall- oder Glasfasern bestehend, bestückt ist. Dabei werden Feststoffe, beispielsweise Abrieb aus den Anlagenteilen oder während der Hydroformylierungsreaktion, den Entspannungsschritten und der Destillation gebildete, in der organischen Flüssigkeit unlösliche Verbindungen zurückgehalten. Die von den Feststoffen gereinigte Lösung wird anschließend über die Leitung (24) abgeführt. Es hat sich nämlich gezeigt, dass derartige, in der organischen Lösung vorhandene Feststoffe schädlich wirken und die Desaktivierung und Zersetzung des Rhodiumkatalysators fördern.
Der über die Leitung (24) abgeführte flüssige Stoffstrom wird anschließend aufgetrennt und über Leitungen (25) und (26) abgeführt. Der über Leitung (25) entnommene Stoffstrom wird auf eine weitere Destillationskolonne (26), die so genannte Nachlaufkolonne gegeben, in der noch flüchtige Aldehydkondensationsprodukte am Kopf über Leitung (27), hochsiedende Aldehydkondensationsprodukte, die noch eine gewisse Flüchtigkeit aufweisen, über den unteren Seitenabzug (28) und ein rhodiumhaltiger, flüssiger Sumpf über die Leitung (29) abgeführt werden. Die über die Leitungen (27) und (28) abgeführten Stoffströme werden ausgeschleust und gegebenenfalls weiter thermisch behandelt. Bei der Kolonne (26) handelt es sich ebenfalls um eine übliche Destillationskolonne. Der über Leitung (29) abgezogene Sumpf wird auf eine Filtriereinrichtung (30) gegeben, die ähnlich wie die Filtriereinrichtung (23) aufgebaut ist und zur Abtrennung vorhandener Feststoffe dient. Die von den Feststoffen befreiten rhodiumhaltigen hochsiedenden Aldehydkondensationsprodukte werden über Leitung (31) befördert. Ein Teil dieses Flüssigkeitsstromes wird über die Leitung (32a) abgeführt und enthaltene Restmengen an Rhodium werden separat aufgearbeitet. Der andere rhodiumhaltige Flüssigkeitsstrom wird über die Leitung (32b) zurückgeführt und mit der über die Leitung (26) herangeführten rhodiumhaltigen Lösung vereinigt und über die Leitung (33) in den Vorratsbehälter (34) geleitet.

Frische organische Phosphor(III)-Verbindung wird über die Leitung (35) in den Vorratsbehälter (34) zu den vereinigten rhodiumhaltigen Flüssigkeitsströmen gegeben.

In den Mischbehälter (36) werden über die Zugabe (37) frische Rhodiumverbindungen, vorzugsweise frisches Rhodium-2-ethylhexanoat, und über die Leitung (38) ein organisches Lösungsmittel, vorzugsweise der gewünschte reine Aldehyd, gegeben.

Die Mischung wird über die Leitung (39) dem Vorratsbehälter (34) zugeführt und die dort hergestellte Flüssigkeit über Leitung (40) abgeführt. In einer bevorzugten Ausgestaltung des erfindungsgemäßen Verfahrens wird die im Mischbehälter (36) angesetzte frische Rhodiumlösung nicht in den Vorratsbehälter (34) gegeben sondern über die Leitung (41), (gestrichelt dargestellt) mit der über Leitung (40) herangeführten, rhodiumhaltigen und mit der organischen Phosphor(III)-Verbindungen versetzten Lösung vereinigt. Die so eingestellt Lösung wird anschließend über die Leitung (42) in den Hochdruckhydroformylierungsreaktor (3) zurückgeführt.

Der Katalysatorkreislauf stammt aus dem Vorratsbehälter (34) und wird über die Leitung (40) dem Reaktor (3) zugeführt. Frisches Rhodium wird über die Leitung (39) oder vorzugsweise über die Leitung (41) herangeführt. Zur Aufrechterhaltung der gewünschten Raum-Zeit-Ausbeute in der Hydroformylierungsreaktion muss ein vergleichsweise hoher Katalysatorkreislauf gefahren werden. So kann die über Leitung (40) herangeführte Flüssigkeitsmenge (Katalysatorkreislauf) das bis zu fünfhundertfache der über Leitung (41) zugeführten Flüssigkeitsmenge (Frischrhodiumeinsatz) betragen, ausgedrückt als Massenverhältnis. Schließlich vereinigen sich die Stoffströme aus den Leitungen (40) und (41) und gehen gemeinsam über Leitung (42) in den Reaktor (3).

Die Menge des über Leitung (39) oder Leitung (41) zugesetzten Rhodiums richtet sich nach der über Leitung (32a) aus dem Prozess ausgeschleusten Rhodiummenge, wobei das Massenverhältnis von zugesetztem frischem Rhodium zu ausgeschleustem Rhodium von 3:1 bis 1: 3 beträgt, bevorzugt 1:1.

Das erfindungsgemäße Verfahren kann auf olefinisch ungesättigte Verbindungen beliebiger Struktur angewandt werden. Dementsprechend sind als Ausgangsmaterial geeignet sowohl Olefine mit innenständiger als auch mit endständiger Doppelbindung und ebenso geradkettige oder verzweigte Olefine. Überdies können die Olefine auch noch durch funktionelle Gruppen substituiert sein, insbesondere solche, die im Verlauf der Reaktion nicht verändert werden. Auch mehrfach olefinisch ungesättigte Verbindungen kommen als Einsatzstoffe in Betracht. Besonders bewährt hat sich das Verfahren bei der Hydroformylierung olefinisch ungesättigter Kohlenwasserstoffe mit 3 bis 6 Kohlenstoffatomen im Molekül, vorzugsweise Propylen und Buten-1 und Gemische enthaltend Buten-1 und Buten-2.

Die Erfindung wird in dem nachfolgenden Beispiel näher erläutert, jedoch nicht auf die beschriebenen Ausführungsformen beschränkt.

### Beispiel 1

### 1. Reaktionsstufe

In einem Reaktor wird unter intensivem Rühren bei einem Synthesegasdruck (CO : H₂ = 1 : 1) von 5 MPa und bei einer Temperatur von 122°C aus Rhodiumacetat und Triphenylphosphintrisulfonat-Na (TPPTS) der aktive, wasserlösliche Katalysator HRhCO(TPPTS)₃ hergestellt. Rhodiumverbindung und TPPTS werden in einer solchen Menge eingesetzt, daß die Rhodiumkonzentration in der wässrigen Katalysatorlösung 300 Gew.-ppm und das Molverhältnis von Rhodium zu Phosphor etwa 1 : 100 beträgt.

Über einen Verteilerring am Boden des Reaktors leitet man vorgewärmtes Propylen in die Reaktionszone und setzt dort das Olefin mit Kohlenmonoxid und Wasserstoff bei 122°C und 5 MPa um. Der aus gasförmigen und flüssigen Bestandteilen bestehende Produktstrom wird dem oberen Teil des Reaktors entnommen und einem Phasentrenner zugeführt, in dem die Trennung der wässrigen Katalysatorlösung vom rohen organischen Reaktionsprodukt und vom Produktabgas erfolgt. Das Produktabgas wird mit dem Reaktorabgas zum Abgasstrom vereinigt. Reaktorabgas wird dem Reaktor entnommen, um eine Anreicherung von Inerten im Gasgemisch zu vermeiden, das im Kreis geführt wird. Der abgeführte Abgasstrom wird auf eine Temperatur von unterhalb 25°C gekühlt, wobei sich ein flüssiges Kondensat, das Abgaskondensat abscheidet, das im Wesentlichen aus Propylen und Propan besteht. Der Wassergehalt im Abgaskondensat beträgt etwa 2 Gew.-%.

### 2. Reaktionsstufe

Das erhaltene Abgaskondensat aus der ersten Reaktionsstufe wird über ein handelsübliches Koalisierfilter mit einer Belastung von 0,5 V/V·h geleitet, wobei ein Druckverlust von 20 hPa beobachtet wird. Das sich dabei abscheidende Wasser wird in einem Gefäß aufgefangen. Nach Austritt durch das Koalisierfilter weist das flüssige Abgaskodensat einen Wassergehalt von noch 0,5 Gew.-% auf.

Das so gereinigte flüssige Abgaskondensat und die bei der Abscheidung des flüssigen Abgaskondensats angefallene gasförmige Phase der ersten Reaktionsstufe dient unter anderem als Einsatz für die zweite Reaktionsstufe.

Dieses Einsatzgemisch wird gegebenenfalls durch Wasserstoff und/oder Kohlenmonoxid und/oder Propylen ergänzt und zusammen mit der Katalysatorlösung auf 21 MPa komprimiert und dem zweiten Reaktor zugeführt. Der Katalysator besteht aus hochsiedenden Aldehydkondensationsprodukten, in denen Triphenylphosphin (TPP) und die Rhodiumkomplexverbindung HRhCO (TPP)₃ gelöst sind. Das Einsatzgemisch wird vom Boden her in den Reaktor eingeführt und hat folgende typische Zusammensetzung (alle Angaben in Gew.-%).

| | |
|---|---|
| Wasserstoff | 2,20 |
| Kohlenmonoxid | 29,94 |
| Kohlendioxid | 0,31 |
| Inerte | 1,29 |
| Propylen | 40,01 |
| Propan | 10,19 |
| n-Butyraldehyd | 7,60 |
| Isobutyraldehyd | 0,61 |
| Butanole | 1,27 |
| C8-Komponenten | 1,60 |
| > C8-Komponenten | 2,76 |
| Triphenylphosphin | 1,21 |
| Triphenylphosphinoxid | 0,50 |
| Wasser | 0,50 |
| Schwefel | Spuren |

Das Molverhältnis von Phosphor zu Rhodium beträgt etwa 80 : 1. Die Umsetzung der Reaktanten erfolgt bei 132°C. 95 - 97% des eingesetzten Propylens (d. h. des nicht umgesetzten Propylens der ersten Reaktionsstufe und gegebenenfalls dem Abgasstrom vor Eintritt in den zweiten Reaktor zugesetztes Propylen) werden in Aldehyd umgewandelt.

Der den zweiten Reaktor verlassende Produktstrom wird zunächst in einen Entgasungsbehälter (Hochdruckabscheider) bei einem Druck geleitet, der dem Druckabfall zwischen dem zweiten Reaktor und dem Entgasungsbehälter entspricht. Die in dem Entgasungsbehälter anfallende Gasphase wird als Hochdruckabgas in einer solchen Menge abgeführt, dass sich in dem Abgas des nachgeschalteten ersten Entspannungsgefäßes (Mitteldruckabscheider) ein Kohlenmonoxidgehalt von 18 Volumenprozent einstellt. Die im Hochdruckabscheider anfallende Flüssigkeit wird in dem Mitteldruckabscheider auf einen Druck von 1 MPa entspannt. Die Gasphase aus dem Mitteldruckabscheider wird in einem Kondensator gekühlt, wobei sich eine flüssige Phase abscheidet und ein Abgas bildet, das als Mitteldruckabgas abgeführt wird und das einen Kohlenmonoxidgehalt von 18 Volumenprozent aufweist. Dieses Abgas wird mit dem Abgas aus dem Hochdruckabscheider vereinigt und aus dem Prozess ausgeschleust. Die im Mitteldruckabscheider anfallende Flüssigkeit wird zusammen mit dem flüssigen Kondensat in einem zweiten Entspannungsgefäß (Niederdruckabscheider) auf einen Druck von 0,2 MPa entspannt.

Die dabei anfallende Gasphase wird kondensiert, nicht kondensierte Anteile als Niederdruckabgas ausgeschleust und die flüssigen Anteile zusammen mit der im Niederdruckabscheider anfallenden flüssigen Phase vereinigt. Das erhaltene rohe n/i-Butyraldehydgemisch wird zur Abtrennung von Rhodium und den hochsiedenden Aldehydkondensationsprodukten auf eine handelsübliche Destillationskolonne gegeben. Bei einer Sumpftemperatur von 110 bis 130°C und Normaldruck wird das n/i-Butyraldehydgemisch abgezogen und in einer weiteren Destillationskolonne in n- und iso-Butyraldehyd aufgespalten. Der ausgeschleuste rhodiumhaltige Destillationssumpf wird über ein als Filtriereinrichtung wirkendes Glasfasergeflecht geleitet, um Feststoffe abzutrennen. Die vom Feststoff befreite Flüssigkeit wird in zwei Teilströme im Massenverhältnis von 90 : 10 aufgeteilt. Der massenmäßig geringere Teilstrom wird auf eine weitere Kolonne (Nachlaufkolonne) gegeben, die bei einer Temperatur von 130°C und bei einem Druck von 10 hPa betrieben wird. Das flüchtige Kopfprodukt und der schwerer flüchtige Seitenabzug, die im Wesentlichen aus hochsiedenden Aldehydkondensationsprodukten bestehen, werden ausgeschleust. Der Sumpfabzug, der im Wesentlichen ebenfalls aus hochsiedenden Aldehydkondensationsprodukten besteht und in dem die Rhodiumkomplexverbindungen gelöst vorliegen, wird nach Überleiten über eine Filtriereinrichtung mit einem Glasfasergeflecht in zwei Teilströme aufgeteilt.

Ein Teilstrom wird ausgeschleust und seine Menge wird so bemessen, dass die über diesen Teilstrom dem Prozess entnommene Rhodiummenge der Rhodiummenge entspricht, die als frische Rhodiumverbindung dem Prozess zugeführt wird.

Der nicht ausgeschleuste Teilstrom wird mit dem nicht über die Nachlaufkolonne geführten Teilstrom zum Katalysatorkreislaufstrom vereinigt.

In einem separaten Behälter wird frisches Rhodium-2-ethylhexanoat mit reinem n-Butyraldehyd vermischt und diese Mischung dem Katalysatorkreislaufstrom in einer solchen Menge zugesetzt, dass die Menge des frisch zugesetzten Rhodiums der zuvor über die hochsiedenden Aldehydkondensationsprodukte ausgeschleusten Rhodiummenge entspricht. Das Massenverhältnis von Katalysatorkreislaufstrom zu der zugesetzten Lösung an frischem Rhodium beträgt etwa 500 : 1. Durch Zugabe von frischem Triphenylphosphin wird ein Molverhältnis von Rhodium zu Phosphor von 1 : 80 eingestellt.

Die so eingestellte rhodiumhaltige Lösung wird anschließend in den zweiten Reaktor zurückgeführt.

Durch die gezielte Einstellung des Kohlenmonoxidgehalts in dem abgeführten Abgas des Mitteldruckabscheiders und durch die genaue Einstellung der ausgeschleusten Rhodiummenge zur frisch zugesetzten Rhodiummenge kann der spezifische Rhodiumverbrauch in der Hydroformylierung von Propylen deutlich reduziert werden und beträgt nach dem erfindungsgemäßen Verfahren 0,5 bis 3 Gew.-ppm frisches Rhodium, bezogen auf Propylen. Arbeitet man hingegen nach dem aus EP 0 805 138 A1 bekannten Stand der Technik, liegt die Zugabe von frischem Rhodium bei 3 bis 6 ppm, bezogen auf Propylen.

## Patentansprüche

1. Verfahren zur Hydroformylierung olefinisch ungesättigter Verbindungen, wobei die Reaktion in einer ersten Reaktionsstufe in einem heterogenen Reaktionssystem unter Verwendung einer wässrigen Lösung, wasserlösliche organische Phosphor(III)-Verbindungen in komplexer Bindung enthaltender Rhodiumverbindungen als Katalysatoren bei Drücken von 0,4 bis 10 MPa erfolgt und Abgas gebildet wird, und wobei das Abgas der ersten Reaktionsstufe einer zweiten Reaktionsstufe zugeführt wird, in der im Abgas noch vorhandene Restmengen der olefinisch ungesättigten Verbindungen in einem homogenen Reaktionssystem in Gegenwart von Komplexverbindungen des Rhodiums mit organischen Phosphor(III)-Verbindungen als Katalysatoren bei Drücken von 15 bis 40 MPa umgesetzt werden, **dadurch gekennzeichnet, dass** man
a) den Austrag der zweiten Reaktionsstufe in einen Entgasungsbehälter bei gleichem oder geringerem Druck leitet, wobei eine Auftrennung in eine Flüssigphase und in eine Gasphase, die als Abgas abgeführt wird, erfolgt; und
b) die in dem Entgasungsbehälter erhaltene Flüssigphase in einer ersten Entspannungsstufe auf einen Druck entspannt, der niedriger als der Druck in dem Entgasungsbehälter ist, wobei eine Auftrennung in eine Flüssigphase und in eine Gasphase erfolgt und wobei die Gasphase unter Bildung eines Abgases kondensiert wird, das abgeführt wird und in dem der Kohlenmonoxidgehalt mehr als 10 Volumenprozent beträgt; und
c) die in der ersten Entspannungsstufe erhaltene Flüssigphase in einer zweiten Entspannungsstufe auf einen Druck entspannt, der niedriger als der Druck der ersten Entspannungsstufe ist, wobei eine Auftrennung in eine Flüssigphase und in eine Gasphase erfolgt und wobei die Gasphase unter Bildung eines Abgases kondensiert wird, das abgeführt wird; und
d) die in der zweiten Entspannungsstufe erhaltene Flüssigphase auf eine Destillationskolonne gibt, wobei eine Auftrennung erfolgt in eine Flüssigphase, die im Wesentlichen hochsiedende Nebenprodukte der Hydroformylierung, den homogen gelösten Rhodiumkatalysator und geringe Mengen an Hydroformylierungsprodukt enthält, und in eine Gasphase, die das Hydroformylierungsprodukt enthält, und
e) aus der Destillationskolonne bei einer Sumpftemperatur von höchstens 150°C einen flüssigen rhodiumhaltigen Austrag abführt, der teilweise ausgeschleust und teilweise in die zweite Reaktionsstufe zurückgeführt wird, und
f) dem in die zweite Reaktionsstufe zurückgeführten flüssigen rhodiumhaltigen Austrag frisches Rhodium in einer solchen Menge zusetzt, dass das Massenverhältnis von zugesetztem frischen Rhodium zu ausgeschleustem Rhodium von 3 :1 bis 1 : 3, bevorzugt 1:1, beträgt.

2. Verfahren gemäß Anspruch 1, **dadurch gekennzeichnet, dass** in der ersten Entspannungsstufe auf einen Druck von 0,1 bis 3,0 MPa, insbesondere 0,5 bis 2,0 MPa, entspannt wird.

3. Verfahren gemäß Anspruch 1, **dadurch gekennzeichnet, dass** in der zweiten Entspannungsstufe auf einen Druck von 0,1 bis 0,5 MPa, insbesondere 0,1 bis 0,3 MPa, entspannt wird.

4. Verfahren gemäß einem oder mehreren der Ansprüche 1 bis 3, **dadurch gekennzeichnet, dass** das abgeführte Abgas der ersten Enspannungsstufe einen Kohlenmonoxidgehalt von 15 bis 40 Volumenprozent, insbesondere 18 bis 30 Volumenprozent, aufweist.

5. Verfahren gemäß einem oder mehreren der Ansprüche 1 bis 5, **dadurch gekennzeichnet, dass** der aus der Destillationskolonne abgeführte flüssige rhodiumhaltige Austrag über ein Filter geleitet wird.

6. Verfahren gemäß einem oder mehreren der Ansprüche 1 bis 5, **dadurch gekennzeichnet, dass** der aus der Destillationskolonne abgeführte flüssige rhodiumhaltige Austrag, der ausgeschleust wird, auf eine zweite Destillationskolonne geben wird, die bei einer Temperatur von 120 bis 150°C und bei einem Druck von 5 bis 15 hPa betrieben wird.

7. Verfahren gemäß Anspruch 6, **dadurch gekennzeichnet, dass** der rhodiumhaltige Sumpfablauf der zweiten Destillationskolonne über ein Filter geleitet wird.

8. Verfahren gemäß einem oder mehreren der Ansprüche 1 bis 7, **dadurch gekennzeichnet, dass** ein Teil des rhodiumhaltigen Sumpfabzugs der zweiten Destillationskolonne ausgeschleust wird und der nicht ausgeschleuste Teil mit dem in die zweite Reaktionsstufe zurückgeführten flüssigen rhodiumhaltigen Austrag der Destillationskolonne vereinigt wird.

9. Verfahren gemäß einem oder mehreren der Ansprüche 1 bis 8, **dadurch gekennzeichnet, dass** man dem in die zweite Reaktionsstufe zurückgeführten flüssigen rhodiumhaltigen Austrag eine organische Phosphor(III)-Verbindung in einer solchen Menge zusetzt, dass das molare Verhältnis von Rhodium zu Phosphor 1 : 3 bis 1: 200, vorzugsweise 1 : 30 bis 1 : 150, beträgt.

10. Verfahren gemäß einem oder mehreren der Ansprüche 1 bis 9, **dadurch gekennzeichnet, dass** man frisches Rhodium in Form von Rhodium-2-ethylhexanoat zuführt.

11. Verfahren gemäß der Ansprüche 5 oder 7, **dadurch gekennzeichnet, dass** als Filter Glasfasern oder Metallfasern verwendet werden.

## Claims

1. A process for the hydroformylation of olefinically unsaturated compounds, where the reaction is carried out, in a first reaction stage, in a heterogeneous reaction system using an aqueous solution containing rhodium compounds containing complexed water-soluble organic phosphorus(III) compounds as catalysts at pressures of from 0.4 to 10 MPa to form an offgas and the offgas from the first reaction stage is fed to a second reaction stage in which the residual amounts of olefinically unsaturated compounds still present in the offgas are reacted in a homogeneous reaction system in the presence of complexes of rhodium with organic phosphorus(III) compounds as catalysts at pressures of from 15 to 40 MPa, **characterized in that**
a) the output from the second reaction stage is passed into a degassing vessel at the same or lower pressure, resulting in separation into a liquid phase and a gas phase which is discharged as offgas; and
b) the liquid phase obtained in the degassing vessel is depressurized in a first depressurization stage to a pressure which is lower than the pressure in the degassing vessel, resulting in separation into a liquid phase and a gas phase, and the gas phase is condensed to form an offgas which is discharged and in which the carbon monoxide content is more than 10 percent by volume; and
c) the liquid phase obtained in the first depressurization stage is depressurized in a second depressurization stage to a pressure which is lower than the pressure of the first depressurization stage, resulting in separation into a liquid phase and a gas phase, and the gas phase is condensed to form an offgas which is discharged; and
d) the liquid phase obtained in the second depressurization stage is introduced into a distillation column, resulting in separation into a liquid phase which contains essentially high-boiling by-products of the hydroformylation, the homogeneously dissolved rhodium catalyst and small amounts of hydroformylation product and a gas phase which contains the hydroformylation product, and
e) a liquid rhodium-containing output is discharged from the distillation column at a temperature at the bottom of not more than 150°C and is partly discharged and partly recirculated to the second reaction stage, and
f) fresh rhodium is added to the liquid rhodium-containing output recirculated to the second reaction stage in such an amount that the mass ratio of added fresh rhodium to discharged rhodium is from 3:1 to 1:3, preferably from 1:1.

2. The process as claimed in claim 1, **characterized in that** depressurization to a pressure of from 0.1 to 3.0 MPa, in particular from 0.5 to 2.0 MPa, is carried out in the first depressurization stage.

3. The process as claimed in claim 1, **characterized in that** depressurization to a pressure of from 0.1 to 0.5 MPa, in particular from 0.1 to 0.3 MPa, is carried out in the second depressurization stage.

4. The process as claimed in one or more of claims 1 to 3, **characterized in that** the offgas discharged from the first depressurization stage has a carbon monoxide content of from 15 to 40 percent by volume, in particular from 18 to 30 percent by volume.

5. The process as claimed in one or more of claims 1 to 5, **characterized in that** the liquid rhodium-containing output from the distillation column is passed through a filter.

6. The process as claimed in one or more of claims 1 to 5, **characterized in that** the liquid rhodium-containing output from the distillation column, which is discharged, is fed to a second distillation column which is operated at a temperature of from 120 to 150°C and a pressure of from 5 to 15 hPa.

7. The process as claimed in claim 6, **characterized in that** the rhodium-containing bottoms from the second distillation column are passed through a filter.

8. The process as claimed in one or more of claims 1 to 7, **characterized in that** part of the rhodium-containing bottoms from the second distillation column is discharged and the undischarged part is combined with the liquid rhodium-containing output from the distillation column which is recirculated to the second reaction stage.

9. The process as claimed in one or more of claims 1 to 8, **characterized in that** an organic phosphorus(III) compound is added to the liquid rhodium-containing output which is recirculated to the second reaction stage in such an amount that the ratio of rhodium to phosphorus is from 1:3 to 1:200, preferably from 1:30 to 1:150.

10. The process as claimed in one or more of claims 1 to 9, **characterized in that** fresh rhodium is introduced in the form of rhodium 2-ethylhexanoate.

11. The process as claimed in claim 5 or 7, **characterized in that** glass fibers or metal fibers are used as filter.

## Revendications

1. Procédé d'hydroformylation de composés oléfiniquement insaturés, la réaction ayant lieu dans un premier étage de réaction dans un système de réaction hétérogène en utilisant une solution aqueuse de composés du rhodium contenant des composés organiques solubles dans l'eau du phosphore (III) en liaison complexe comme catalyseurs à des pressions de 0,4 à 10 MPa et un effluent gazeux étant formé, l'effluent gazeux du premier étage de réaction étant alimenté dans un deuxième étage de réaction, dans lequel des quantités résiduelles des composés oléfiniquement insaturés encore présentes dans l'effluent gazeux sont transformées dans un système de réaction homogène en présence de composés complexes du rhodium avec des composés organiques du phosphore (III) comme catalyseurs à des pressions de 15 à 40 MPa, **caractérisé en ce qu'**on
a) fait passer le produit sortant du deuxième étage de réaction dans un récipient de dégazage à la même pression ou à une pression plus faible, une séparation en une phase liquide et en une phase gazeuse, qui est évacuée en tant qu'effluent gazeux, se produisant ; et
b) détend la phase liquide obtenue dans le récipient de dégazage dans un premier étage de détente à une pression qui est inférieure à la pression dans le récipient de dégazage, une séparation en une phase liquide et en une phase gazeuse se produisant et la phase gazeuse étant condensée en formant un effluent gazeux, qui est évacué et dans lequel la teneur en monoxyde de carbone est supérieure à 10% en volume ; et
c) détend la phase liquide obtenue dans le premier étage de détente dans un deuxième étage de détente à une pression qui est inférieure à la pression dans le premier étage de détente, une séparation en une phase liquide et en une phase gazeuse se produisant et la phase gazeuse étant condensée en formant un effluent gazeux, qui est évacué ; et
d) amène la phase liquide obtenue dans le deuxième étage de détente sur une colonne de distillation, une séparation en une phase liquide, qui contient essentiellement des produits secondaires de point d'ébullition élevé de l'hydroformylation, le catalyseur à base de rhodium dissous en phase homogène et de faibles quantités de produit d'hydroformylation, et en une phase gazeuse, qui contient le produit d'hydroformylation, se produisant ; et
e) évacue de la colonne de distillation, à une température du fond d'au plus 150°C, un produit liquide contenant du rhodium, qui est partiellement soutiré et partiellement recyclé dans le deuxième étage de réaction, et
f) ajoute au produit liquide contenant du rhodium recyclé dans le deuxième étage de réaction du rhodium frais en une quantité telle que le rapport massique de rhodium frais ajouté à rhodium soutiré est de 3:1 à 1:3, de préférence de 1:1.

2. Procédé selon la revendication 1, **caractérisé en ce qu'**on détend dans le premier étage de détente à une pression de 0,1 à 3,0 MPa, en particulier de 0,5 à 2,0 MPa.

3. Procédé selon la revendication 1, **caractérisé en ce qu'**on détend dans le deuxième étage de détente à une pression de 0,1 à 0,5 MPa, en particulier de 0,1 à 0,3 MPa.

4. Procédé selon l'une ou plusieurs des revendications 1 à 3, **caractérisé en ce que** l'effluent gazeux évacué du premier étage de détente présente une teneur en monoxyde de carbone de 15 à 40% en volume, en particulier de 18 à 30% en volume.

5. Procédé selon l'une ou plusieurs des revendications 1 à 5, **caractérisé en ce que** le produit liquide contenant du rhodium évacué de la colonne de distillation est guidé sur un filtre.

6. Procédé selon l'une ou plusieurs des revendications 1 à 5, **caractérisé en ce que** le produit liquide contenant du rhodium évacué de la colonne de distillation, qui est soutiré, est amené sur une deuxième colonne de distillation qui est exploitée à une température de 120 à 150°C et à une pression de 5 à 15 hPa.

7. Procédé selon la revendication 6, **caractérisé en ce que** le produit sortant du fond contenant du rhodium de la deuxième colonne de distillation est guidé sur un filtre.

8. Procédé selon l'une ou plusieurs des revendications 1 à 7, **caractérisé en ce qu'**une partie du produit sortant du fond contenant du rhodium de la deuxième colonne de distillation est soutiré et la partie non soutirée est réunie avec le produit liquide contenant du rhodium de la colonne de distillation recyclé dans le deuxième étage de réaction.

9. Procédé selon l'une ou plusieurs des revendications 1 à 8, **caractérisé en ce qu'**on ajoute au produit liquide contenant du rhodium recyclé dans le deuxième étage de réaction un composé organique du phosphore (III) en une quantité telle que le rapport molaire de rhodium à phosphore est de 1:3 à 1:200, de préférence de 1:30 à 1:150.

10. Procédé selon l'une ou plusieurs des revendications 1 à 9, **caractérisé en ce qu'**on alimente du rhodium frais sous forme de 2-éthylhexanoate de rhodium.

11. Procédé selon les revendications 5 ou 7, **caractérisé en ce qu'**on utilise, comme filtre, des fibres de verre ou des fibres métalliques.
